# EUROPÄISCHE PATENTANMELDUNG

(11) **EP 1 321 878 A2**
(43) Veröffentlichungstag der Anmeldung: **25.06.2003**
(21) Anmeldenummer: 02102740.4
(22) Anmeldetag: 12.12.2002
(51) Int. Cl.: G06F 17/60, G06F 19/00

(54) **Patientendatenverarbeitungssystem und -verfahren**

(30) Priorität: 14.12.2001 DE 10161381
(71) Anmelder: Philips Intellectual Property & Standards GmbH, 20099 Hamburg (DE); Koninklijke Philips Electronics N.V., 5621 BA Eindhoven (NL)
(72) Erfinder: Carlsen, Ingwer,Dr., Postfach 50 04 42 52088 Aachen (DE); Lorenz, Cristian, Dr., Postfach 50 04 42 52088 Aachen (DE); Renisch, Steffen Dr., Postfach 50 04 42 52088 Aachen (DE); Schlathölter, Thorsten Dr., Postfach 50 04 42 52088 Aachen (DE); Zonneveld, Frans Prof. Dr., Postfach 50 04 42 52088 Aachen (DE)
(74) Vertreter: Volmer, Georg, Dipl.-Ing.

(57) **Zusammenfassung**

Die Erfindung betrifft ein Patientendatenverarbeitungssystem sowie ein entsprechendes Verfahren zur protokollgesteuerten Verarbeitung von Patientendaten. Um Standard- und Routine-Aktivitäten auf einen Computer zu verlagern und möglichst automatisiert ablaufen zu lassen, wird erfindungsgemäß ein Patientendatenverarbeitungssystem vorgeschlagen mit:
a) einer Patientendatenspeichereinheit zur Speicherung von Patientendaten
b) einer Protokollspeichereinheit zur Speicherung von Standardprotokollen zur automatischen Verarbeitung von Patientendaten,
c) einer Protokollauswahleinheit zur Auswahl eines Standardprotokolls aus der Protokollspeichereinheit anhand der Patientendaten,
d) einer Protokollinstantiierungseinheit zur Umwandlung des ausgewählten Standardprotokolls in ein patientenbezogenes Protokoll anhand der Patientendaten,
e) einer Protokollverarbeitungseinheit zur Verarbeitung des patientenbezogenen Protokolls, und
f) einer Datenverarbeitungseinheit zur Ausführung der Protokollschritte und Ausgabe des Verarbeitungsergebnisses.

## Beschreibung

Die Erfindung betrifft ein Patientendatenverarbeitungssystem zur protokollgesteuerten Verarbeitung von Patientendaten, eine Protokollausführungsvorrichtung, ein Patientendatenverarbeitungsverfahren und ein Computerprogramm zur Umsetzung des Verfahrens.

Konventionelle Workflow-Systeme zur Unterstützung von klinischen Abläufen sind bekannt und werden beispielsweise von der Firma Algotec unter den Bezeichnungen "MediLink" und "MediFlow" im Internet unter der Domain http://www.algotec.com/products/angeboten. Mit diesen Systemen wird erreicht, dass die in einer Klinik anfallenden Informationen, insbesondere Patientendaten, wie persönliche Daten, Befundungsberichte, Bilddaten usw., an die erforderlichen Stellen in der Klinik verteilt werden und an allen notwendigen Geräten elektronisch verfügbar sind. Außerdem können von einzelnen Geräten Statusmeldungen an andere Geräte geschickt werden, so dass eine automatische elektronische Ablaufplanung möglich ist. Die Abläufe innerhalb einzelner Systeme werden jedochnicht von den Workflow-Systemen gesteuert.

Der vorliegenden Erfindung liegt die Aufgabe zugrunde, ein Patientendatenverarbeitungssystem und ein entsprechendes Verfahren anzugeben, mit denen die Abläufe in den zur Befundung dienenden Geräten automatisiert werden können. Diese Aufgabe wird erfindungsgemäß durch ein Patientendatenverarbeitungssystem gemäß Anspruch 1 gelöst, welches ausgestaltet ist mit:
a) einer Patientendatenspeichereinheit zur Speicherung von Patientendaten
b) einer Protokollspeichereinheit zur Speicherung von Standardprotokollen zur automatischen Verarbeitung von Patientendaten,
c) einer Protokollauswahleinheit zur Auswahl eines Standardprotokolls aus der Protokollspeichereinheit anhand der Patientendaten,
d) einer Protokollinstantiierungseinheit zur Umwandlung des ausgewählten Standardprotokolls in ein patientenbezogenes Protokoll anhand der Patientendaten,
e) einer Protokollverarbeitungseinheit zur Verarbeitung des patientenbezogenen Protokolls, und
f) einer Datenverarbeitungseinheit zur Ausführung der Protokollschritte und Ausgabe des Verarbeitungsergebnisses.

Eine einen wesentlichen Bestandteil des erfindungsgemäßen Patientendatenverarbeitungssystems bildende Protokollausführungsvorrichtung ist in Anspruch 7 angegeben. Ein zu dem in Anspruch 1 angegebenen System korrespondierendes Verfahren ist in Anspruch 8 angegeben. Darüber hinaus betrifft die Erfindung auch ein Computerprogramm mit Computerprogrammmitteln zur Umsetzung der Verfahrensschritte des erfindungsgemäßen Verfahrens, wenn dieses auf einem Computer ausgeführt wird, wie es in Anspruch 9 angegeben ist.

Erfindungsgemäß ist vorgesehen, dass die Abläufe in den zur Befundung dienenden Geräten gemäß eines Protokolls gesteuert werden. Es wird also nicht nur, wie bei den bekannten Workflow-Systemen, das Endresultat einer Befundung, z.B. der Befundungsbericht oder erstellte Bilder, elektronisch verwaltet und weitergegeben, sondern die Erstellung des Endresultats wird automatisiert. Dazu werden geeignete Einheiten als Teil des Patientendatenverarbeitungssystems vorgeschlagen, die in der Lage sind, ein geeignetes Protokoll auszuwählen, anhand der Patientendaten des gerade zu befundenden Patienten zu personalisieren, zu interpretieren und auszuführen. Damit wird ein weiterer Schritt in der Systemautomatisierung erreicht, der weit über die Fähigkeiten der bekannten Workflow-Systeme hinausgeht. Während diese nämlich lediglich eine Patientendatenspeichereinheit, beispielsweise eine klinische IT-Infrastruktur, und Datenverarbeitungseinheiten, z.B. medizinische Workstations, aufweisen, sind erfindungsgemäß zusätzlich eine Protokollspeichereinheit, eine Protokollauswahleinheit, eine Protokollinstantiierungseinheit und eine Protokollverarbeitungseinheit vorgesehen, um die gewünschte Protokollauswahl und -ausführung vorzunehmen.

Vorteilhafte Ausgestaltungen sind in den Unteransprüchen angegeben. So ist beispielsweise vorgesehen, dass die Patientendatenspeichereinheit eine Bildspeichereinheit zur Speicherung von patientenbezogenen medizinischen Bilddaten und ein klinisches Informationssystem zur Speicherung patientenbezogener Informationen aufweist. Darüber hinaus kann die Datenverarbeitungseinheit eine Bildverarbeitungseinheit, eine Bilddarstellungseinheit und eine Bedienschnittstelle aufweisen.

Erfindungsgemäß soll die Auswahl des auszuführenden Protokolls aus der Vielzahl von Standardprotokollen automatisch erfolgen. Dazu werden die vorhandenen Patientendaten ausgewertet, insbesondere die Patientenvorgeschichte wie beispielsweise Alter, Geschlecht, frühere Krankheiten, vermutete Krankheit und/oder medizinische Bilddaten des Patienten. Die Protokollauswahl erfolgt dabei aufgrund einer Reihe von Bedingungen und kann beispielsweise durch eine entsprechendes Expertensystem durchgeführt werden.

Bevorzugt ist vorgesehen, dass die Standardprotokolle Protokollschritte zur automatischen Bildverarbeitung, Bilddarstellung, Bilderfassung und/oder Befunderstellung aufweisen. Es sollen also so viel wie möglich Schritte automatisiert werden.

In einer weiteren Ausgestaltung ist eine Workflow-Einheit vorgesehen zur Steuerung der zu bearbeitenden Patientendaten bzw. der zu bearbeitenden Patienten.

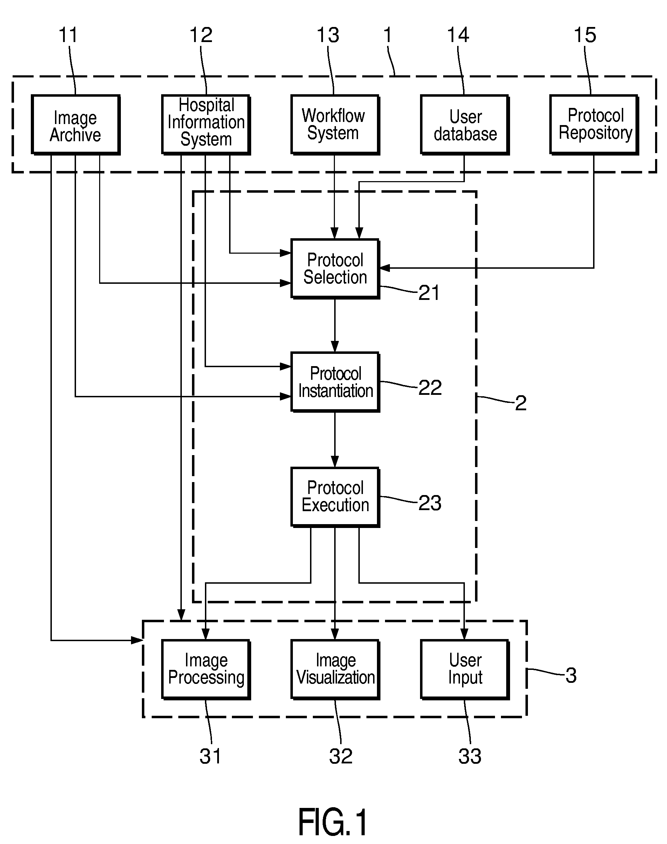

Die Erfindung wird nachfolgend anhand der einzigen Figur erläutert, die ein Blockschaltbild eines erfindungsgemäßen Patientendatenverarbeitungssystems zeigt. Der erste Schritt bei der erfindungsgemäßen Verarbeitung von Patientendaten, die einerseits in einen Bildspeicher (Image archive 11) in Form von medizinischen Bilddaten von Patienten und andererseits in Form von sonstigen patientenbezogenen Informationen in einem klinischen Informationssystem (Hospital Information System) 12 gespeichert sind, besteht darin, dass eine Workflow-Einheit (Workflow-System) 13 die Bearbeitung eines neuen Falls, z.B. die Erstellung eines Befunds für einen neuen Patienten, anfordert (Case-Request). Diese Anforderung wird an eine oder mehrere medizinische Arbeitsstationen geschickt, die erfindungsgemäß eine Protokollvorrichtung 2 und eine Datenverarbeitungseinheit 3 aufweist. Wird der Fall von einer Arbeitsstation bzw. dem daran arbeitenden Benutzer, beispielsweise einem daran arbeitenden medizinischtechnischen Assistenten oder einem Radiologen, akzeptiert, wird zunächst von einer Protokollauswahleinheit (Protocol selection) 21 ein Standardprotokoll aus einer Reihe von Standardprotokollen, die in einer Protokollspeichereinheit (Protocol repository) 15 gespeichert sind, automatisch ausgewählt. Aufgrund der vorliegenden Patientendaten, wie insbesondere der Patientenvorgeschichte (Alter, Geschlecht, Verdacht auf..., usw.), der Art der aufgenommenen Bilder etc,. erfolgt dabei die Auswahl des geeigneten Standardprotokolls. Insbesondere erfolgt die Protokollauswahl aufgrund einer Reihe von Bedingungen und wird bevorzugt durch ein entsprechendes Expertensystem ausgeführt.

Nach Auswahl des Protokolls muss dieses instantiiert werden. Das bedeutet, dass die Platzhalter in dem Standardprotokoll, die auf Patientendaten verweisen, z.B. auf Bilddaten, persönliche Daten, Befunde, etc., aufgelöst und mit den auf den aktuell bearbeiteten Patienten zutreffenden, tatsächlich vorliegenden Patientendaten gefüllt werden müssen. Dies erfolgt in der Protokollinstantiierungseinheit (Protocol instantiation) 22, die auf die in den Einheiten 11 und 12 gespeicherten Patientendaten zugreift. Es muss also die zur Ausführung des Protokolls abstrakte Funktionalität auf die reale Funktionalität der ausführenden medizinischen Arbeitsstation abgebildet werden. Dieser Vorgang liefert ein auf der Arbeitsstation ausführbares Protokoll, das beispielsweise in einer gängigen Computer-Skript-Sprache formuliert ist.

Danach wird dieses Protokoll in einer Protokollverarbeitungseinheit 23 ausgeführt, d.h., Schritt für Schritt wird die entsprechende Verarbeitungs-, -Visualisierungs- oder Interaktionsfunktionalität in der Datenverarbeitungseinheit 3, die dazu entsprechend eine Bildverarbeitungseinheit (Image processing) 31, eine Bilddarstellungseinheit (Image visualization) 32 und eine Bedienschnittstelle (User input) 33 aufweist, durchgeführt.

Das erfindungsgemäße automatische Patientendatenverarbeitungsverfahren wird vorzugsweise für den vollständigen Prozess der Diagnose und Befunderstellung benutzt, z.B. in einer Radiologieabteilung in einem Krankenhaus. Erforderlich ist dafür eine adäquate medizinische IT-Infrastruktur, insbesondere medizinische Arbeitsstationen, ein geeignetes Netzwerk, ein Radiologieinformationssystem und ein elektronisches Bilddatenarchiv. Das Verfahren kann für jede beliebige Bildgebungsmodalität wie Computertomographie, Magnetresonanztomographie, Ultraschall, Röntgen sowie andere klinische Datenerfassungsverfahren wie Elektrokardiographie angewendet werden.
Durch das erfindungsgemäße Verfahren kann die Effizienz der bildgestützten medizinischen Diagnose und Befunderstellung deutlich verbessert werden, indem Standardprozesse automatisiert werden. Dies führt zu einem Anstieg der Kosteneffizienz und zu einer schnelleren Erstellung von Befunden. Auch das Qualitätsmanagement wird verbessert aufgrund der Möglichkeit des automatischen Datenverarbeitungssystems, Standardverfahren einzusetzen und Prüfschritte einzubauen, um beispielsweise die Vollständigkeit von Berichten oder Daten zu prüfen. Zusätzlich wird auch die Effizienz von Datenspeicherung und Datenverarbeitung gesteigert, da vorzugsweise ausschließlich elektronische Dokumente und Verfahren benutzt werden.

Ein konkreter Beispielfall, bei dem Verdacht auf ein Aneurysma im Willis-Kreis ("Circle of Willis") besteht. Für die Diagnose soll ein kranialer CTA-Scan der Schädelbasis gemacht werden. Das ausgewählte Protokoll betrifft dabei die Nachverarbeitung dieses Scans.

In einem ersten Protokollschritt wird zunächst ein patientenbezogenes Koordinatensystem definiert. Dazu werden mit einem Bilddarstellungssystem, z.B. mit einem Ortho-Viewer drei orthogonale Ansichten der erfassten Bilddaten dargestellt. Der Benutzer wird aufgefordert, die mittlere saggitale Ebene durch drei oder mehr Punkte, die Links-Rechts-Achse mit zwei dedizierten Markern im Innenohr und die Vorne-Hinten-Achse durch zwei dedizierte Marker in der Schädelbasis festzulegen.

In einem zweiten Schritt wird ein Teil-Volumen, das rekonstruiert werden soll, automatisch definiert. Dabei wird das definierte Koordinatensystem benutzt, um die Koordinaten der Eckpunkte eines quaderförmigen interessierenden Volumens, das den Willis-Kreis enthält, zu berechnen. In einem dritten Schritt wird anschließend das quaderförmige Volumen zusammen mit den Bilddaten in dem Ortho-Viewer dargestellt. Dem Benutzer bietet sich dabei die Möglichkeit, den ausgewählten Quader beizubehalten oder manuelle Adjustierungen vorzunehmen.

In einem vierten Schritt, der optional ist, kann halbautomatisch eine Entfernung von Knochenstrukturen innerhalb des selektierten Teilvolumens erfolgen. Dazu wird der Benutzer gefragt, ob für eine verbesserte Visualisierung der Bildvoxel, die Knochenstrukturen enthalten, eine automatische Markierung erfolgen soll, um diese Bildvoxel von nachfolgenden Visualierungen auszunehmen. Das Ergebnis dieser automatischen Operation wird vom Benutzer entweder bestätigt, manuell korrigiert oder verworfen. Nachfolgend werden in einem fünften Schritt Maximum-Intensitäts-Projektionen (MIP) erstellt, die entlang des Patientenkoordinatensystems orientiert sind Dazu werden bevorzugt drei Projektionen des selektierten Teilvolumens entlang der drei senkrecht aufeinander stehenden Raumrichtungen erstellt.

In einem sechsten Schritt wird ein Satz von schaufelradförmigen Maximum-Intensitäts-Projektionen des Teilvolumens erstellt. Dabei ist die Schaufelradachse entlang der Links-Rechts-Patientenachse orientiert. Vor der Erstellung der Projektionen kann der Benutzer auch gefragt werden, ob die für die Erzeugung des Satzes der Projektionen benutzten Parameter, wie beispielsweise Winkelinkrement und Anzahl der Projektionen, akzeptiert oder geändert werden soll. Optional kann in einem siebten Schritt ein geeignetes Drucklayout selektiert werden, wo mit von den erzeugten Projektionen dann Filmabzüge erstellt werden können.

In einem achten Schritt kann ein geeignetes Muster für einen Befundbericht, vorliegend zB. das Muster "CTA Willis-Kreis, Verdacht auf Aneurysma" selektiert werden. Darin werden automatisch die demographischen Patientendaten eingsfügt und der Satz der erstellten Projektionen wird dem Bericht angähängt. Schließlich werden basierend auf den erzeugten Projektionen die Diagnose und der Befundbericht erstellt.

Wie oben erläutert, erfolgt vorliegend eine Aufgabenteilung für Standardfälle. Beispielsweise werden die erläuterten Schritte 1 bis 8 von einem technischen Assistenten und Schritt 9 von einem Radiologen durchgeführt, wobei natürlich in schwierigen Fällen der Radiologe auch bei den Schritten 1 bis 8 involviert sein kann. Schließlich kann auch ein nochmaliger Durchlauf des Verfahrens erfolgen, wenn das Ergebnis nicht zufriedenstellend ist.

Die Auswahl und der Ablauf des Protokolls ist stark davon abhängig, wie die Patientenhistorie aussieht oder welche besonderen Situationen in dem aktuellen Fall gerade vorliegen. Beispielsweise kann bei Vorliegen eines zwei Jahre alten CTA-Scans des Patienten das oben erläuterte Protokoll so geändert werden, dass nach Schritt 6 ein automatischer Registrierungsschritt eingefügt wird. Dabei wird der neue CTA-Scan mit dem alten vorliegenden CTA-Scan automatisch registriert. Beide Scans sowie eine Overlay-Darstellung der beiden Scans kann dann in dem Ortho-Viewer dargestellt werden. Auf Anforderung können auch zusätzliche Projektionen für den alten Datensatz erstellt werden.

Die klinischen Verfahren, die durchgeführt werden, hängen sehr stark von dem Untersuchungskontext ab. Für verschiedene Patientenhistorien und Scan-Verfahren werden unterschiedliche Protokolle verwendet. Außerdem werden die Protokolle vor der Abarbeitung an die speziellen Patientengegebenheiten oder auch an spezielle Besonderheiten des Krankenhauses oder des Personals angepasst. Im folgenden sollen verschiedene Gegebenheiten anhand einzelner Beispiele erläutert werden, die für die Auswahl und Konkretisierung eines Protokolls relevant sein können:
a) der spezielle Fall: Fallhistorie, z.B. Verdacht auf Schlaganfall;
b) das Scan-Verfahren: die abgebildete anatomische Region, die Anwendung von Kontrastmitteln etc., z.B. CT-Scan mit Kontrastmittel;
c) der Patient: Geschlecht, Alter, Gewicht, etc., z.B. Patient A;
d) das Krankenhaus: lokale Gegebenheiten, Schulen, z.B. allgemeines Krankenhaus B;
e) die Benutzerfunktion: Arbeitsteilungsaspekte, z.B. Assistent, Radiologe;
f) die Person des Benutzers: personelle Gegebenheiten oder Vorzüge, z.B. Radiologe C;
g) die Arbeitsstation: unterstützte Funktionalitäten, Beschränkungen, z.B. Werkstation D.

Zu Beginn einer Untersuchung wird ein Protokoll aus der Protokolldatenbank entsprechend den oben beschriebenen Kontextinformationen ausgewählt. Das Protokoll definiert, an welchem Punkten ein bestimmter Status erreicht ist, der an andere Einheiten der IT-Infrastruktur kommuniziert werden muss, beispielsweise die Information, dass die Datenvorbereitung erfolgt ist, dass der Bericht signiert wurde usw. Durch diese Art der Kommunikation ermöglicht das Protokoll, die Bearbeitungslisten des involvierten Personals zu beeinflussen, um so eine Reduzierung von Verzögerungen zwischen nachfolgenden Arbeiten zu erreichen.

Das Protokoll selbst schreibt die durchzuführende Aktivität sowie die dazugehörigen Parameter und notwendigen Benutzerinteraktionen vor. Ein wichtiger Aspekt des protokollgesteuerten Verfahrensablaufes ist die Arbeitsteilung zwischen den verschiedenen involvierten Personen, den zugehörigen Verantwortlichkeiten und Rechten. So ist beispielsweise ein Assistent berechtigt, ein Protokoll für einen Befundbericht auszuwählen und spezielle Patientendaten einzugeben, aber er hat keine Befugnis, die Diagnose einzufügen oder den Bericht zu signieren. Derartige Berechtigungen können ebenfalls in den Standardprotokollen enthalten sein.

Die folgende Liste gibt einen Überblick über allgemeine Tätigkeiten, die potentiell protokollautomatisiert durchgeführt werden können:
- Bildnachverarbeitungsschritte, wie beispielsweise Rauschreduzierung oder Resampling;
- Bildsegmentierung, beispielsweise Knochenentfernung oder Rotationsachsenbestimmung von röhrenförmigen Strukturen;
- Definition von patientenbezogenen Positionen, Orientierungen oder Koordinatensystemen, wie beispielsweise der mittleren saggitalen Ebene;
- Definition des interessierenden Bereichs, beispielsweise Orientierung, Position und Dicke von Teilvolumina;
- Selektion von Visualisierungsverfahren, beispielsweise MPR, MIP, mIP, CVP, DVR oder virtuelle Endoskopie, und der zugehörigen Parametersätze, beispielsweise Level und Fenster;
- Definition und Erstellung von dedizierten Sätzen von Renderings, z.B. für eine Schaufelrad-Rekonstruktion;
- Definition eines Darstellungsprotokolls;
- Filmlayout und Ausdruck;
- Auswahl eines elektronischen Berichtsmusters und Einfügung von einfachen Einträgen, beispielsweise Patienten und Scan-Informationen oder Standardbetrachtungen;
- Archivierung von Daten;
- Verschicken von Benachrichtigungen.

## Patentansprüche

1. Patientendatenverarbeitungssystem zur protokollgesteuerten Verarbeitung von Patientendaten mit:
a) einer Patientendatenspeichereinheit (11,12) zur Speicherung von Patientendaten
b) einer Protokollspeichereinheit (15) zur Speicherung von Standardprotokollen zur automatischen Verarbeitung von Patientendaten,
c) einer Protokollauswahleinheit (21) zur Auswahl eines Standardprotokolls aus der Protokollspeichereinheit (15) anhand der Patientendaten,
d) einer Protokollinstantiierungseinheit (22) zur Umwandlung des ausgewählten Standardprotokolls in ein patientenbezogenes Protokoll anhand der Patientendaten,
e) einer Protokollverarbeitungseinheit (23) zur Verarbeitung des patientenbezogenen Protokolls, und
f) einer Datenverarbeitungseinheit (3) zur Ausführung der Protokollschritte und Ausgabe des Verarbeitungsergebnisses.

2. Patientendatenverarbeitungssystem nach Anspruch 1,
**dadurch gekennzeichnet,**
**dass** die Patientendatenspeichereinheit (11,12) eine Bildspeichereinheit (11) zur Speicherung von patientenbezogenen medizinischen Bilddaten und ein klinisches Informationssystem (12) zur Speicherung patientenbezogener Informationen aufweist.

3. Patientendatenverarbeitungssystem nach Anspruch 1,
**dadurch gekennzeichnet,**
**dass** die Datenverarbeitungseinheit (3) eine Bildverarbeitungseinheit (31), eine Bilddarstellungseinheit (32) und eine Bedienschnittstelle (33) aufweist.

4. Patientendatenverarbeitungssystem nach Anspruch 1,
**dadurch gekennzeichnet,**
**dass** die Protokollauswahleinheit (21) ausgebildet ist zur Auswahl eines Standardprotokolls anhand der als Patientendaten gespeicherten Patientenvorgeschichte und/oder medizinischer Bilddaten des Patienten.

5. Patientendatenverarbeitungssystem nach Anspruch 1,
**dadurch gekennzeichnet,**
**dass** die Standardprotokolle Protokollschritte zur automatischen Bildverarbeitung, Bilddarstellung, Bilderfassung und/oder Berunderstellung aufweisen.

6. Patientendatenverarbeitungssystem nach Anspruch 1,
**dadurch gekennzeichnet,**
**dass** eine Workfloweinheit (13) vorgesehen ist zur Steuerung der zu bearbeitenden Patientendaten bzw. der zu bearbeitenden Patienten.

7. Protokollvorrichtung zur protokollgesteuerten Verarbeitung von Patientendaten mit:
a) einer Protokollauswahleinheit (21) zur Auswahl eines Standardprotokolls aus einer Vielzahl von auf einer Protokollspeichereinheit (15) gespeicherten Standardprotokollen zur automatischen Verarbeitung von Patientendaten anhand von in einer Patientendatenspeichereinheit (11, 12) gespeicherten Patientendaten,
b) einer Protokollinstantiierungseinheit (22) zur Umwandlung des ausgewählten Standardprotokolls in ein patientenbezogenes Protokoll anhand der Patientendaten,
c) einer Protokollverarbeitungseinheit (23) zur Verarbeitung des patientenbezogenen Protokolls, und
d) einer Datenverarbeitungseinheit (3) zur Ausrührung der Protokollschritte und Ausgabe des Verarbeitungsergebnisses.

8. Patientendatenverarbeitungsverfahren zur protokollgesteuerten Verarbeitung von Patientendaten mit den Schritten:
a) Protokollauswahl zur Auswahl eines Standardprotokolls aus einer Vielzahl von auf einer Protokollspeichereinheit (15) gespeicherten Standardprotokollen zur automatischen Verarbeitung von Patientendaten anhand von in einer Patientendatenspeichereinheit (11, 12) gespeicherten Patientendaten,
b) Protokollinstantiierung zur Umwandlung des ausgewählten Standardprotokolls in ein patientenbezogenes Protokoll anhand der Patientendaten,
c) Protokollverarbeitung zur Verarbeitung des patientenbezogenen Protokolls, und
d) einer Datenverarbeitung zur Ausführung der Protokollschritte und Ausgabe des Verarbeitungsergebnisses.

9. Computerprogramm mit Computerprogrammmitteln zur Umsetzung der Verfahrensschritte des Verfahrens nach Anspruch 8, wenn das Verfahren auf einem Computer ausgeführt wird.
